Europäisches Patentamt

European Patent Office

Office européen des brevets

(11) Numéro de publication: **0 462 194 B1**

# FASCICULE DE BREVET EUROPEEN

(45) Date de publication de fascicule du brevet: **08.06.94** (51) Int. Cl.⁵: **A61K 37/02**, A61K 47/36

(21) Numéro de dépôt: **90904852.2**

(22) Date de dépôt: **09.03.90**

(86) Numéro de dépôt internationale :
**PCT/FR90/00164**

(87) Numéro de publication internationale :
**WO 90/10456 (20.09.90 90/22)**

(54) **COMPOSITIONS STABILISEES A BASE DE FGF.**

(30) Priorité: **09.03.89 FR 8903086**

(43) Date de publication de la demande:
**27.12.91 Bulletin 91/52**

(45) Mention de la délivrance du brevet:
**08.06.94 Bulletin 94/23**

(84) Etats contractants désignés:
**AT BE CH DE DK ES FR GB IT LI LU NL SE**

(56) Documents cités:
**EP-A- 0 267 015**

**MERCK INDEX (1983), page 426**

(73) Titulaire: **THERAPEUTIOUES SUBSTITUTIVES**
**Université Paris-Nord, Avenue J.B.Clément**
**F-93430 Villetaneuse(FR)**

(72) Inventeur: **BARRITAULT, Denis**
**4, rue Française**
**F-75001 Paris(FR)**
Inventeur: **JOZEFONVICZ, Jacqueline**
**62, Deuxième Avenue**
**F-60260 Lamorlaye(FR)**
Inventeur: **SLAOUI, Faouzi**
**77, rue de Rome**
**F-75017 Paris(FR)**
Inventeur: **TARDIEU, Michèle**
**14, impasse Diderot**
**F-94500 Champigny-sur-Marne(FR)**
Inventeur: **CARUELLE, Jean-Pierre**
**32, rue Jules-Joffrin**
**F-94100 Saint-Maur(FR)**
Inventeur: **COURTY, José**
**15, allée Verte**
**F-94440 Villecresnes(FR)**

(74) Mandataire: **Ores, Irène et al**
**CABINET ORES**
**6, Avenue de Messine**
**F-75008 Paris (FR)**

EP 0 462 194 B1

**EP 0 462 194 B1**

**Description**

La présente invention est relative à des agents à activité régénératrice cellulaire et tissulaire constitués par des dextranes, à des compositions stabilisées contenant lesdits agents associés à des facteurs de croissance des fibroblastes (FGFs) ainsi qu'à leurs applications *in vitro* telles que stockage des FGFs et cultures cellulaires et *in vivo* comme agent thérapeutique, notamment pour la cicatrisation et la régénération tissulaire ou comme agent cosmétique.

Les facteurs de croissance des fibroblastes (FGFs) ont été mis en évidence par de nombreuses équipes, à la suite des études des activités biologiques des facteurs de croissance, obtenus à partir d'extraits d'un très grand nombre de tissus ou d'organes (cerveau, hypophyse, rétine, humeur vitrée, choroïde, iris, cartilage, rein, foie, placenta, corpus *luteum*, prostate, os, muscle etc...)

La diversité même des tissus étudiés et des cellules stimulées par ces facteurs *in vitro* et *in vivo*, ainsi que le grand nombre d'équipes qui ont indépendamment contribué à la caractérisation, l'isolement et l'identification complète de ces facteurs, explique la multitude de noms et sigles donnés par ces différents auteurs pour les désigner.

Il apparaît que tous ces extraits contiennent des facteurs de croissance de la famille des FGFs et que cette famille peut être divisée en deux branches principales.

La première branche a été décrite sous les noms de FGF basique, de facteur basique de croissance des fibroblastes ou de type 2 affin pour l'héparine ou "Heparin Binding Growth Factor II" (HBGF II), de facteur de croissance dérivé du cerveau ou Brain-Derived Growth Factor (BDGF), de facteur de croissance dérivé de l'oeil ou Eye-Derived Growth Factor II (EDGF II), de facteur de croissance des astrocytes II ou AGF II, de facteur de croissance dérivé du cartilage (CDGF) etc... alors que la deuxième branche de la famille FGF a été décrite sous les noms de FGF acide ou facteur affin pour l'héparine de type I (HBGF I), facteur dérivé du cerveau ou BDGF I, etc...

Ces facteurs ont été nommés soit selon le type de cellules cibles utilisées (facteurs de croissance des fibroblastes, astrocytes ou cellules endothéliales avec les sigles FGF, AGF, ECGF), soit selon la source à partir de laquelle ce facteur est extrait (exemple : facteur de croissance dérivé du cerveau, dérivé de la rétine ou des yeux, du cartilage, d'hépatocytes en culture avec respectivement les sigles BDGF, RDGF, EDGF, CDGF, HDGF...), soit encore selon une propriété biochimique ou biologique (facteurs de croissance affins pour l'héparine (HBGF) ou facteur tumoral angiogénique (TAF) ; les deux principales branches de la famille sont nommées selon ces sigles, précédés ou suivis de acide ou basique, ou type I ou type II.

C'est en suivant l'activité biologique sur des cellules en culture que ces facteurs ont pu être purifiés. Les premières caractéristiques physicochimiques (poids moléculaire et point isoélectrique) ont été publiées dès 1975 (GOSPODAROWICZ, J. Biol. Chem. 250, 2515) pour la forme basique et en 1982 (BARRITAULT et al., J. Neurosci. 8, 477-490) pour la forme acide.

La purification à homogénéité des deux formes du FGF a permis d'établir leurs structures primaires (ESCH. et al., 1985, Proc. Natl. Acad. Sci. US, 82, 6507 pour la forme basique et GIMENEZ G. et al., 1985, Science, 230, 1385-1388 pour la forme acide).

L'isolement des deux formes a été grandement favorisée par la mise en évidence d'une forte affinité de ces facteurs pour l'héparine et l'utilisation subséquente d'une chromatographie d'affinité sur héparine immobilisée (SHING et al., 1984, Science, 223, 1296-1299).

Il est connu que, *in vitro*, les FGFs sont capables de stimuler la prolifération et la différenciation d'un grand nombre de cellules provenant de tissus et d'espèces différents.

On peut citer notamment les cellules fibroblastiques, endothéliales, épithéliales, les kératinocytes, chondrocytes, myoblastes, astrocytes etc..., ainsi que la survie neuronale.

Il est également connu que, *in vivo*, les FGFs présentent des propriétés neurotrophiques, angiogéniques et cicatrisantes.

On peut citer notamment le Brevet français 79 18282, qui enseigne un procédé de stimulation de la croissance des cellules épidermiques ; ce procédé montre en particulier le rôle d'un extrait aqueux de rétine partiellement purifié et contenant du FGF sur la stimulation desdites cellules épidermiques.

On connaît également le Brevet américain US-A-4 477 435, qui enseigne une méthode de cicatrisation de l'épithélium cornéen à l'aide d'une composition contenant un extrait aqueux de rétine.

On connaît également de nombreux travaux concernant la mise en évidence et la caractérisation des FGFs et leur rôle dans la régénération et la cicatrisation de la peau, des vaisseaux, des nerfs, des os, des muscles etc... tant *in vitro* qu'*in vivo*.

On peut citer en particulier le Brevet américain US-A-4 444 760, qui décrit un facteur de croissance acide dérivé du cerveau, son procédé d'extraction et son application à la cicatrisation des plaies et la demande de Brevet européen EP-A-186 084 qui décrit une méthode de stimulation de la croissance des

2

EP 0 462 194 B1

cellules endothéliales vasculaires à l'aide d'une composition contenant le facteur de croissance acide dérivé du cerveau décrit précédemment.

Les FGFs décrits ci-dessus sont obtenus par purification ; on connait par ailleurs, par la Demande internationale PCT US86/01879, des FGFs obtenus par recombinaison génétique.

Une autre composition cicatrisante à base d'au moins un FGF est décrite dans la Demande de Brevet européen EP-A-243 179 et comprend en outre du collagène et de l'héparine et/ou un glycoaminoglycane.

Dans ces différents documents, l'application topique du FGF, seul ou associé, est réalisée à l'aide de formulations usuelles telles que crèmes, pâtes, solutions, gels ou associées à des polymères, des éponges et des pompes permettant un relargage lent des FGFs, comme cela est en particulier décrit dans la demande Internationale PCT US86/01879, dans laquelle il est précisé que des formulations comprenant des FGFs recombinants et des excipients ou des molécules de transport appropriés peuvent être préparées, notamment des lotions, des gels, des formes retard ou des crèmes, lesdites formulations étant éventuellement associées à d'autres principes actifs, tels que des antibiotiques. Les formes retard, décrites dans cette Demande, comprennent en particulier des polymères.

Les compositions obtenues peuvent notamment être utilisées comme cicatrisant, dans le contrôle de la coagulation, dans l'amélioration des dommages neurologiques et dans la régénération des tissus durs.

Il apparaît toutefois, que le FGF ne présente pas systématiquement une stimulation de la cicatrisation ; en effet une absence de stimulation a été notamment rapportée dans J. Dermatol. Sing. Oncol. ; de ce fait, l'application topique de FGF acide ou basique doit souvent être itérative, pour obtenir les effets maximaux, bien que certaines compositions de l'Art antérieur telles que des éponges d'alcoolpolyvinylique imbibées de FGFs, appliquées sous la peau, entraînent une prolifération fibroblastique et myoblastique.

Ceci est dû à une instabilité thermique de la molécule, à une inactivation de la molécule liée au pH, à une protéolyse par des enzymes, ainsi qu'à une interaction entre les FGFs et les glycoaminoglycanes comme l'héparane sulfate ou le protéohéparane sulfate des membranes cellulaires ou des membranes basales, entraînant une immobilisation des FGFs pouvant empêcher leur accès aux récepteurs cellulaires.

De tels inconvénients limitent les possibilités de stockage et d'exploitation des FGFs.

La Demande de Brevet européen EP-A-267 015 a proposé, pour pallier cet inconvénient, une composition contenant un facteur de croissance polypeptidique, plus particulièrement de l'EGF, et une quantité de polysaccharide soluble dans l'eau suffisante pour stabiliser ledit facteur contre la perte d'activité biologique, en présence d'eau notamment. Il est spécifié dans cette Demande, que les polysaccharides solubles dans l'eau qui peuvent être utilisés incluent les dérivés de la cellulose, l'amidon , l'agar, l'acide alginique, la gomme arabique, les dextranes, les fructanes, l'inuline, les mannanes, les xylanes, les arabinanes, les chitosanes, le glycogène et les glucanes.

Les Inventeurs, poursuivant leurs travaux sur les dextranes, ont mis en évidence de nouvelles propriétés de dextranes substitués fonctionnalisés ; lesdits dextranes s'avèrent présenter une activité régénératrice cellulaire et tissulaire propre et présentent, de plus, non seulement une action stabilisante sur une composition de FGF mais coopèrent avec le FGF dans l'activité biologique de ce dernier.

La Demanderesse s'est en conséquence donné pour but de pourvoir à un agent à activité régénératrice cellulaire et tissulaire ainsi qu'à des compositions contenant ledit agent associé à des FGFs qui répondent mieux aux nécessités de la pratique que les compositions visant au même but proposées dans l'Art antérieur, notamment en ce que les compositions conformes à l'invention ont une stabilité nettement améliorée, permettant un stockage plus aisé et de ce fait un effet thérapeutique supérieur aux compositions de l'Art antérieur et en ce que leur fréquence d'application est ainsi nettement diminuée.

La présente invention a pour objet l'utilisation d'au moins un dextrane substitué fonctionnalisé par des fonctions choisies dans le groupe qui est constitué par le carboxyméthyle, le benzylamide et le benzylamide sulfonate, pour l'obtention d'un médicament destiné à une activité thérapeutique de régénération cellulaire et tissulaire.

Conformément à cette utilisation, les dextranes substitués fonctionnalisés sont choisis dans le groupe qui comprend des dextranes solubles et des dextranes insolubles.

On entend par dextranes solubles substitués fonctionnalisés, ceux qui sont notamment décrits dans le Brevet français n° FR-A-2 555 589 ou dans le Brevet français n° FR-A-2 461 724.

On entend par dextranes insolubles substitués fonctionnalisés, ceux qui sont notamment décrits dans la Demande de Brevet français n° 82 01641 (FR-A-2. 555 589) ou dans le Brevet français n° 2 461 724.

De tels dextranes sont stables et ne perdent pas leurs propriétés avec le temps.

De plus, ils présentent la propriété inattendue de présenter une activité régénératrice cellulaire et tissulaire propre, à faibles doses et plus particulièrement une activité cicatrisante.

La présente invention a également pour objet une composition stabilisée, caractérisée en ce qu'elle comprend un agent à activité régénératrice cellulaire et tissulaire tel que défini ci-dessus associé avec au

3

moins un FGF acide et/ou un FGF basique et/ou un dérivé et/ou un analogue et/ou un fragment de ceux-ci présentant une activité biologique, lequel agent est capable à la fois de restaurer partiellement ou totalement l'activité biologique du/des facteurs FGF acides et/ou basiques inactivés par un stockage de longue durée ou la température et de coopérer avec le FGF dans l'activité biologique de ce dernier.

Une telle composition présente une activité de régénération cellulaire et tissulaire, et notamment une action cicatrisante, accrue par rapport aux compositions de l'Art antérieur.

Selon un mode de réalisation avantageux de la composition conforme à l'invention, celle-ci comprend de 0,1 à 1 000 µg/ml d'au moins un agent à activité régénératrice cellulaire et tissulaire tel que défini ci-dessus et de 0,01 ng/ml à 300 µg/ml d'au moins un FGF choisi dans le groupe constitué par les FGF acides, les FGF basiques, leurs dérivés, leurs analogues et leurs fragments présentant une activité biologique.

Conformément à l'invention, les compositions stabilisées contenant l'agent à activité régénératrice cellulaire et tissulaire seul ou associé à au moins un FGF peuvent être associés à d'autres principes actifs et/ou à au moins un véhicule pharmaceutiquement acceptable et/ou à un support physiologiquement acceptable.

Les principes actifs associés sont choisis dans le groupe qui comprend notamment les anesthésiques locaux, les antiinfectieux, des protéines sériques et du collagène.

On peut citer notamment, comme anesthésique local, la lidocaïne, comme substances bactériostatiques, les sels de sodium, les sels d'argent, leurs dérivés ou des sulfadiazines. On peut citer comme antibiotique, la streptomycine ; on peut citer comme protéine sérique, la sérumalbumine ou la fibronectine ; on peut citer également des collagènes solubles et de l'élastine.

Conformément à l'invention, lorsque le véhicule est de l'eau, ladite composition est en outre associée à des tampons et/ou à des sels appropriés, de manière à maintenir le mélange approximativement à un pH compris entre 6,8 et 7,4 et à une force ionique comprise par exemple entre 0,1 et 0,2 en équivalent NaCl.

De telles associations conformes à l'invention sont ci-après dénommées "composition matrice".

Selon un mode de réalisation avantageux de ladite composition, elle est associée à des liposomes.

De telles compositions conformes à l'invention sont ci-après dénommées composition FGF-dextrane fonctionnalisé-liposome et composition dextrane fonctionnalisé-liposome.

Selon un autre mode de réalisation avantageux de ladite composition, le support est choisi dans le groupe qui comprend notamment les pansements et les biomatériaux.

Selon un autre mode de réalisation de la composition conforme à l'invention, celle-ci est sous forme d'aérosol, lorsque le véhicule est un gaz approprié.

La composition "matrice" est avantageusement appliquée directement en solution ou en aérosol.

Conformément à l'invention, ladite composition, notamment ladite composition "matrice" est incluse dans une forme galénique telle que pommade, crème, pâte, lotion ou imprègne un gel, notamment un gel de collagène.

Egalement conformément à l'invention, ladite composition, notamment ladite composition "matrice" est incluse et/ou imprègne un support approprié tel que pansement ou biomatériau favorisant directement ou indirectement la réparation cellulaire (par exemple, un fil de suture chirurgical ou le corail pour greffe osseuse).

Ladite composition "matrice" peut notamment être incluse dans des crèmes ou lotions utilisées traditionnellement, notamment les crèmes à base de lanoline telles que "SILVEADENE", "MARIO", "AQUAPHOR", "EQUALIA", pour des applications sur la peau ; elle peut également être incluse ou imbiber des pansements tels que pansements de textiles, de tissus synthétiques ou d'éponges, ou des produits naturels servant de recouvrement de plaies, par exemple des gels de collagène ou des dermes d'origine animale.

Ladite composition "matrice" conforme à l'invention, doit imprégner ces différentes formes de pansements, de manière à ce que le facteur FGF et/ou le dextrane fonctionnalisé substitué puissent être en contact ou diffuser, jusqu'aux tissus cibles.

La composition conforme à l'invention est notamment maintenue sur le site de la blessure et sur les blessures ouvertes, de manière à maintenir l'hydratation selon les techniques de l'homme de l'Art, particulièrement élaborées dans le domaine des greffes de peau.

Les pansements occlusifs peuvent être imprégnés de la même manière, adsorbés ou recouvrir un support naturel ou synthétique.

Pour les applications sur les cornées, le véhicule doit être compatible avec la tolérance oculaire (par exemple, le produit commercialisé sous le nom de "LACRIBULE"), des solutions salines, des solutions isotoniques (par exemple, le "NEOCADRON" (Merck-Sharp-Dohme).

Ces véhicules peuvent contenir également des agents conservateurs comme les chlorures de benzyl-diméthyl alcoyle d'ammonium ou l'éthylène diamine tétraacétate de sodium (EDTA).

Conformément à l'invention, la composition FGF-dextrane fonctionnalisé-liposome ou la composition dextrane fonctionnalisé-liposome est incluse dans une forme galénique telle que pommade, crème, pâte, lotion ou imprégne un gel, notamment un gel de collagène.

Dans le cas de dextranes fonctionnalisés insolubles, ceux-ci peuvent également être inclus seuls ou associés au FGF dans des supports comme les crèmes, les gélatines ou gels de collagènes, ou sur des fibres synthétiques ou naturelles, supports habituels de pansements de recouvrement. L'inclusion des polymères fonctionnalisés insolubles peut se faire par addition de solution de collagène et gélification. Les procédures décrites dans une série de brevets de YANNAS peuvent être utilisées. Dans ces brevets (US-A-4, 060081), une composition laminaire composite donnant une peau équivalente dans laquelle la partie en contact avec la blessure est couverte de collagène réticulé avec un glycosaminoglycane, le mélange étant obtenu par addition de glycosaminoglycanes au collagène solubilisé, l'ensemble étant précipité ou réticulé par du glutaraldéhyde (Brevet US-A-4418691).

La composition conforme à l'invention, est notamment préparée par le mélange d'au moins un FGF approprié avec au moins un agent à activité régénératrice et stabilisante.

Lesdits FGFs sont obtenus par extraction et purification, à partir de sources naturelles, par synthèse chimique ou bien par des techniques de recombinaison génétique appropriées.

Lesdits FGFs sont d'origine humaine ou bien proviennent d'autres animaux, notamment d'autres mammifères.

De nombreux procédés de purification pour extraire et isoler les deux formes de FGFs à partir de ces sources naturelles (rétine, cerveau, hypophyse, placenta, rein etc...) ont été décrits dans l'Art antérieur.

Les méthodes préférées de la présente invention sont celles décrites dans Biochimie, 1986 (COURTY et al.) ou celle décrite dans la Demande de Brevet français 2 613 936, qui utilise la chromatographie d'affinité sur polystyrènes substitués biospécifiques.

Ces méthodes préférées incluent une étape de traitement de l'extrait tissulaire à pH très acide, excluant ainsi tout risque de contamination virale, et l'utilisation d'une chromatographie sur héparine immobilisée ou polystyrène substitué.

Les deux formes de FGF peuvent ainsi être isolées et séparées, avec les autres protéines ou individuellement avec un degré de pureté suffisant pour être débarassé de quantités significatives d'autres matériels contaminants.

Outre les dispositions qui précèdent, l'invention comporte encore d'autres dispositions, qui ressortiront de la description qui va suivre, qui se réfère à des exemples de mise en oeuvre du procédé objet de la présente invention ainsi qu'à des exemples montrant l'effet des dextranes substitués fonctionnalisés sur la protection de l'activité biologique des FGFs.

Il doit être bien entendu, toutefois, que ces exemples sont donnés uniquement à titre d'illustration de l'objet de l'invention, dont ils ne constituent en aucune manière une limitation.

### Exemples

### Exemple 1 : Procédé de stabilisation des FGF.

1) Préparation d'un dextrane substitué fonctionnalisé (agent de régénération cellulaire et tissulaire).

. 30 grammes de dextrane T40 (0,185 mole) sont dissous dans 146 ml d'eau distillée et refroidis à 4°C dans un bain de glace fondante. 59,2 g de NaOH (1,48 moles) sont dissous dans 100 ml d'eau distillée, puis refroidis à 4°C. La solution de soude est versée lentement dans la solution de dextrane sous agitation et l'ensemble est maintenu à 4°C pendant 20 minutes. On ajoute alors 61 g de $ClCH_2COOH$ (0,647 mole) très progressivement, afin que la température atteigne 20°C après 5 minutes. Le milieu réactionnel est alors porté à 40°C en 10 minutes et maintenu à cette température pendant 90 minutes, puis refroidi vers 20°C. Le pH est abaissé vers 7 avec de l'acide acétique concentré. L'ensemble est précipité dans 2 litres de méthanol, filtré et lavé deux fois avec 1 litre d'éthanol, puis séché sous vide à 40°C.

. 10 g du polymère modifié précédent sont dissous dans 55 ml d'eau distillée acidifiée à pH 3. 60 ml de diméthylformamide sont ajoutés très progressivement sous agitation, en maintenant le pH à la valeur de 3. La température est abaissée à -15°C et 12,3 ml de N-méthylmorpholine sont ajoutés avec 14,5 ml de chloroformate d'isobutyle. On ajoute ensuite 12,2 ml de benzylamine. Après 30 minutes, le polymère est précipité dans 800 ml de méthanol filtré et séché.

. 9 g du polymère modifié précédent sont dispersés dans 25 ml de dichlorométhane anhydre. Un mélange de 0,26 ml de HSO$_3$Cl et de 2,5 ml de dichlorométhane est ajouté dans le réacteur et l'ensemble est maintenu 4 heures à température ambiante. Après filtration et lavage par du dichlorométhane, le produit est séché, dissous dans 30 ml d'eau et le pH est ajusté à la valeur 7,0. La solution est ultrafiltrée contre une solution tampon, puis contre de l'eau distillée. La solution est alors lyophilisée jusqu'à obtention du polymère sec.

Une autre méthode de préparation d'un dextrane substitué fonctionnalisé peut être utilisée comme telle que celle décrite dans le Brevet Européen n° 0 023 854.

2) Préparation du/des FGF.
- On traite le/les extraits cellulaires pendant une nuit en présence d'acide acétique à pH 3, puis on sépare les FGFs par chromatographie sur héparine immobilisée ou polystyrène substitué.

3) Préparation d'une composition stable de FGF conforme à l'invention.
- On prépare une solution de dextrane à partir du polymère sec obtenu en 1) en le mettant en solution dans un tampon phosphate isotonique (PBS), de manière à obtenir une concentration de 400 $\mu$g/ml.
- On met les FGFs extraits en 2) en solution dans ce tampon contenant les dextranes substitués appropriés, de manière à obtenir une concentration en FGFs de 100 $\mu$g/ml.

**Exemple 2 : Pommade stabilisée conforme à l'invention.**

| | |
|---|---|
| FGF | 10 $\mu$g |
| DF | 5 mg |
| Carboxyméthyl cellulose | 2,5 g |
| Eau purifiée stérile apyrogène | 100 ml |
| DF = Dextrane fonctionnalisé de type E tel que défini dans le tableau III ci-après. | |

La crème obtenue peut être appliquée sur une plaie de type scarification sur un rat, et ce pendant trois jours.

**Exemple 3 : Pansement stabilisé conforme à l'invention.**

Le support du pansement est constitué d'un film collagène "Pangil" des Laboratoires FOURNIER, imprégné par adsorption passive d'un mélange de FGF et de Dextrane fonctionnalisé dans les proportions suivantes :

| | |
|---|---|
| FGF | 10 $\mu$g |
| DF | 500 $\mu$g |
| Sérum physiologique | 10 ml |

Après incubation du film de collagène pendant 30 minutes à 4°C dans la solution décrite ci-dessus, on obtient un pansement pouvant être utilisé dans des cas d'ulcérations diverses, de plaies superficielles ou profondes.

Ce pansement peut être stocké sous vide et empaqueté.

\* ETUDE DE L'EFFET DE POLYMERES BIOSPECIFIQUES FONCTIONNALISES SUR LA PROTECTION DE L'ACTIVITE BIOLOGIQUE DES FGFs *in vitro.*

**Méthodologie utilisée pour la mesure de l'activité biologique des FGFs *in vitro.***

Les méthodes d'évaluation *in vitro* de l'activité biologique des FGFs sont décrites dans de nombreuses publications, et sont toutes basées soit sur une mesure de l'augmentation du nombre de cellules induite par des doses croissantes de facteurs ajoutés au milieu de culture des cellules, soit sur une augmentation de l'incorporation de thymidine tritiée dans le DNA des cellules stimulées par le facteur de croissance. Dans les deux méthodes évoquées, ces augmentations sont dépendantes de la dose de facteur ajoutée, et l'on peut donc établir des effets dose et des courbes dose-réponse avec un effet maximal de stimulation. Par simplification, on définit une unité de stimulation comme la dose de facteur de croissance qui, ajoutée à un millilitre de milieu de culture sur des cellules cibles est capable d'induire une augmentation du nombre de cellules ou de l'incorporation de thymidine tritiée correspondant à la moitié (50 %) de la valeur maximale de cette augmentation mesurée dans la courbe dose-réponse. Cette définition et la reproductibilité de ces mesures sont exposées notamment dans PLOUET et al., 1984, Cellular and Molecular Biology 30, p 105.

## EXEMPLE A : EFFET PROTECTEUR DU DEXTRAN SUBSTITUE CONTRE L'INACTIVATION DES FGFs ACIDE ET BASIOUE PAR DES pH ACIDES ET ALCALINS.

Dans ces expériences, les FGFs sont en solution à une concentration de 100 $\mu$g par millilitre, dans un tampon phosphate isotonique (PBS) ne contenant pas de dextrane (témoin) ou contenant du dextrane substitué à 400 $\mu$g/ml. 10 $\mu$l de ces différentes solutions sont prélevés et mélangés soit à 1 ml de tampon PBS, d'acide acétique ($CH_3COOH$) dilué ajusté à pH 2 (environ 1 N), soit de soude (NaOH) diluée ajustée à pH 9,0. Ces échantillons sont incubés à 20°C pendant deux heures et un prélèvement de 1 $\mu$l est effectué pour le dosage de l'activité biologique.

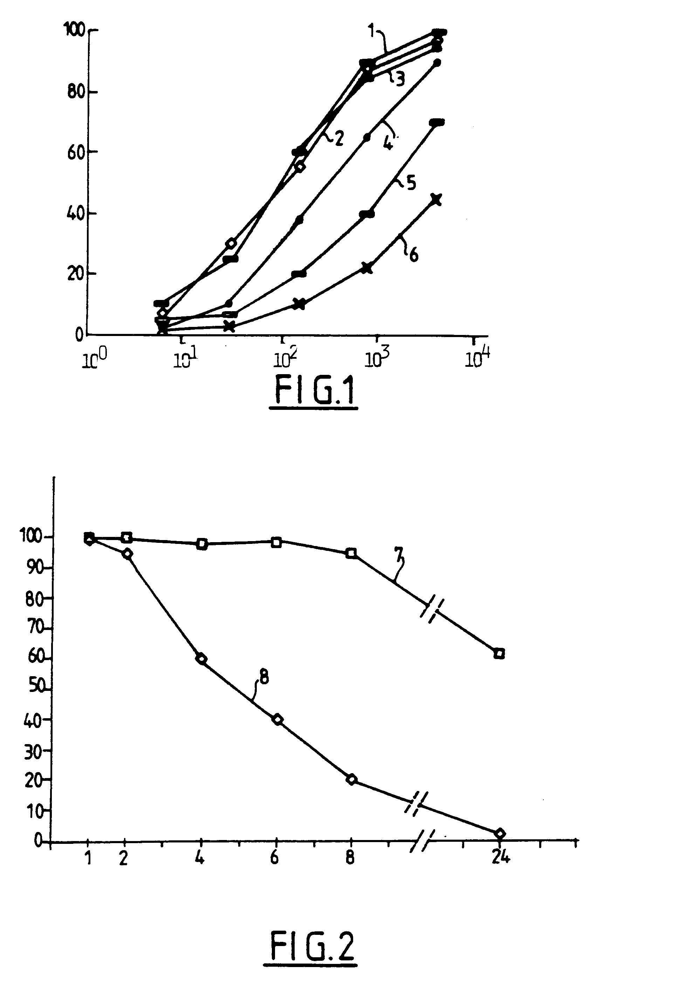

La figure 1 présente la courbe dose-réponse du bFGF sur des fibroblastes CCL39.

Cette figure comporte en abscisse le logarithme de la concentration de bFGF en pg/ml et en ordonnée la pourcentage de stimulation.

La courbe 1 correspond au témoin ; la courbe 2 correspond au bFGF seul à pH 2 ; la courbe 3 correspond au bFGF en présence de dextrane à pH 2 ; la courbe 4 correspond au bFGF en présence de dextrane à pH 9 ; la courbe 5 correspond au bFGF seul à pH 9 et la courbe 6 correspond au témoin en présence de dextrane.

L'augmentation d'incorporation de thymidine tritiée représente la valeur du nombre de coups par minute (cpm) obtenue au plateau de la courbe dose-réponse du bFGF seul moins la valeur en cpm de thymidine tritiée incorporée dans les cellules en absence de FGF et déterminée dans la même expérience.

Les courbes 3 et 4 montrent que le bFGF en présence de dextrane conserve son pouvoir de stimulation tant en milieu acide que basique.

Le tableau I résume les résultats obtenus avec les FGFs acide et basique. L'unité de stimulation est arbitrairement fixée à 1 pour le aFGF ou le bFGF de départ, incubé deux heures à 20°C.

TABLEAU I

|  | pH 2 | pH 7 | pH 9 |
|---|---|---|---|
| FGFb (0°C) |  | 0,9 |  |
| FGFb (2 H, 20°C) | 53 | 1 | 13 |
| FGFb + DF (2H, 20°) | 1 | 1 | 2,5 |
| FGFb + H.S. (2H, 20°) | 3 | 1 | 4 |
| FGFa (0°) |  | 1 |  |
| FGFa (2H, 20°) | 6 | 1 | 6 |
| FGFa + DF (2H, 20°) | 0,5 | 0,4 | 2 |
| FGFa + H.S. (2H, 20°) | 1,5 | 0,8 | 4,5 |

DF = dextrane fonctionnalisé : dans cet exemple, il s'agit du dextrane E tel que défini dans le tableau III, ci-après.

H.S. = heparane sulfate : (Société BIOVALORIS à Plouhermel (Ile-et-Villaine, FRANCE)).

Ce tableau montre l'effet protecteur du DF (Dextrane fonctionnalisé) contre l'inactivation des FGFs acide et basique induits par des pH acides et alcalins.

L'incubation du FGF basique pendant deux heures à 20°C dans une solution tampon pH 2 à 9 induit une inactivation de l'activité biologique du FGF basique.

En effet, il faut 53 fois plus de produit à pH acide et 13 fois plus à pH basique pour induire un effet biologique au produit initial.

L'adjonction de DF à ce mélange protège totalement l'activité biologique du FGF basique contre des incubations à pH 2 ou 9.

Des résultats similaires sont observés dans le cas du FGF acide concernant les deux types de traitement.

**EXEMPLE B : EFFET DU DEXTRANE FONCTIONNALISE (DF) SUR L'INACTIVATION DES FGFs PAR LA TEMPERATURE A COURT TERME ET LONG TERME.**

Dans cet exemple, le FGF préparé comme dans l'exemple A est incubé à 4°C, 20°C, 37°C ou 60°C pendant différents temps, en l'absence ou en présence de 400 $\mu$g de dextrane fonctionnalisé (DF) tel que défini dans le tableau III ci-après, puis dosé.

Les résultats sont présentés dans le tableau II ci-après.

TABLEAU II

|  | 4°C | 20°C | 37°C | 60°C |
|---|---|---|---|---|
| bFGF t = 0' | 1 |  |  |  |
| bFGF t = 30' | 1 | 1 | 3,5 | > 100 |
| bFGF + DF t = 30' | 1 | 1 | 1 | 9 |
| aFGF t = 0' | 1 |  |  |  |
| aFGF t = 30' | 1 | 1 | 2 | > 100 |
| aFGF + DF t = 30' | 0,4 | 0,4 | 0,4 | 5 |
| bFGF t = 24 H | 1 | 1 | 6 |  |
| bFGF + DF t = 24 H | 1 | 1 | 1 |  |
| aFGF | 1 | 1 | 1 |  |
| aFGF + DF | 0,4 | 0,4 | 0,4 |  |
| bFGF t = 7 jours | 2 | 5 | >100 |  |
| bFGF + DF t = 7 jours | 1 | 1 | 1 |  |
| bFGF + H.S. t = 7 jours | 1 | 2 | 6 |  |
| aFGF t = 7 jours | 2,5 | 8 | >100 |  |
| aFGF + DF t = 7 jours | 0,4 | 0,4 | 3 |  |
| DF = dextrane fonctionnalisé | | | | |
| H.S. = héparane sulfate | | | | |

L'unité de stimulation initiale est fixée arbitrairement à une valeur égale à 1.

Dans ce tableau, on observe une forte inhibition de l'activation du FGF acide ou basique induit par un traitement d'une semaine à 37°C. La présence du DF dans le milieu d'incubation protège les deux types de FGF contre la dénaturation thermique.

Des résultats similaires sont observés en utilisant l'HS (Héparane Sulfate), équivalent biologique du DF.

**EXEMPLE C : EFFET DE DIFFERENTS DEXTRANES FONCTIONNALISES SUR LES EFFETS DOSE-REPONSE DU FGF.**

L'effet de différents dextranes fonctionnalisés est mesuré à l'unité sur le tableau III ci-dessous.

TABLEAU III

| Dérivés dextranes | % D | % W | % X | % Y | R/us |
|---|---|---|---|---|---|
| A | 100 | 0 | 0 | 0 | 1 |
| B | 0 | 106 | 0 | 0 | 1,6 |
| C | 0 | 84 | 21 | 0 | 1,7 |
| D | 10 | 76 | 0 | 14 | 2,6 |
| E | 0 | 89 | 6 | 5 | 2,36 |
| F | 0 | 74 | 16 | 10 | 3,1 |
| G | 65 | 30 | 1 | 4 | 2,54 |
| H | 29 | 42 | 24 | 5 | 2,1 |

Pourcentage :

D : Dextrane

W : Carboxyméthyle

X : Benzylamide

Y : Benzylamidesulfonate

R/us est la valeur du rapport des valeurs des unités de stimulation du aFGF sans dextrane fonctionnalisé divisé par l'unité de stimulation en présence de dextrane fonctionnalisé.

* ETUDE DE L'EFFET DE POLYMERES BIOSPECIFIQUES FONCTIONNALISES SUR LA PROTECTION DE L'ACTIVITE BIOLOGIQUE DES FGFS *in vivo*

**Exemple D : ETUDES CINETIQUE, PLANIMETRIQUE ET HISTOLOGIQUE DE L'EFFET CICATRISANT DE L'ASSOCIATION FGF/DEXTRANE FONCTIONNALISE.**

Protocole expérimental :

Les opérations sont réalisées sur des rats Wistar mâles de 300 à 400 grammes. Chaque expérience est effectuée sur une série de 5 animaux.

Types de plaies :

Deux types de plaies cutanées sont effectuées sur la face dorsale des animaux, préalablement rasée.
- Les prélèvements sont effectués par punch (0,6 cm de diamètre) jusqu'au plancher musculaire.
- Des scarifications de 1 cm de long sont pratiqués au scalpel. Elles n'affectent pas la région dermoépidermique.

Mode opératoire :

Selon le type de plaies, les blessures sont traitées par différents mélanges de produits en solution dans du sérum physiologique tamponné (pH 7,4) stérilisé.

Ces solutions sont déposées pour les plaies par punch dans un tampon collagène (GINGESTAT) prédécoupé aux mesures exactes de l'excision tissulaire.

Dans le cas des scarifications, les produits sont déposés directement sous forme liquide sur la plaie.

Les effets de l'association FGF (basique ou acide ou un mélange en solution de 1 ng à 10 $\mu$g/ml) et des dextranes fonctionnalisés (en solution de 100 ng à 1 mg/ml) sont évalués et comparés à l'action d'un dextrane fonctionnalisé substitué seul et de chacun des constituants, considérés comme témoins de réaction (collagène, solution de dissolution, FGF).

Chaque série expérimentale d'animaux est sacrifiée à intervalle de temps défini par période de 24 heures, et les régions lésées sont prélevées pour deux types d'étude :
- une analyse morphologique externe avec planimétrie de la plaie ;
- une étude histologique.

Résultats :

I - Effets stabilisants des dextranes fonctionnalisés :

Du FGF radiomarqué à l'$^{125}$I est déposé en présence ou en absence de dextrane fonctionnalisé dans un tampon de collagène.

La variation de la radioactivité dans le collagène imprégné est appréciée en fonction du temps.

Les résultats sont illustrés dans la figure 2, qui comporte en abscisse le temps en heure et en ordonnée, le pourcentage de radioactivité. La courbe 7 correspond au FGF en présence de dextrane et la courbe 8 correspond au FGF seul.

La radioactivité est mesurée dans le gel de collagène et dans des prélèvements effectués en périphérie de la plaie, à 2 cm de celle-ci, par un punch équivalent à celui d'origine.

II - Etudes morphologique et histologique :

A) Etude morphologique :

L'observation de l'évolution des plaies à l'oeil nu permet de constater une action très nette de l'association FGF + dextrane fonctionnalisé sur la vitesse et la qualité de la cicatrisation superficielle (épidermisation + lyse du caillot).

1) Après 24 heures, les tampons de collagène imprégnés par cette association ont totalement adhéré aux parois de la plaie, et ne peuvent être prélevés que par lésions des tissus régénérés. Les expérimentations témoins ne manifestent une adhérence totale des collagènes qu'au bout de 36 à 48 heures.

2) La réépithélialisation est visible à l'oeil nu au bout du troisième jour, en présence de l'association FGF + dextrane fonctionnalisé, alors qu'une image identique sur les contrôles demande des temps d'expérimentation de 5 à 7 jours.

3) Analyse planimétrique : l'analyse planimétrique de la surface externe des plaies montre l'absence totale de rétraction des tissus en régénération.

Le taux de rétraction cicatricielle est évalué en fonction du temps en considérant le rapport P/A où P est le périmètre de la plaie et A la surface de la cicatrice.

L'ordre de grandeur de ce rapport P/A est du type K/R où K est une constante et R le rayon de la plaie originelle circulaire.

En fonction du temps, plus ce rapport est faible et constant, plus la cicatrice conserve une planimétrie proche de celle de la lésion d'origine. Par conséquent, plus le rapport P/A est faible, plus le taux de restructuration cicatriciel est limité. La qualité de cicatrisation peut ainsi être reflétée par l'absence de contraction.

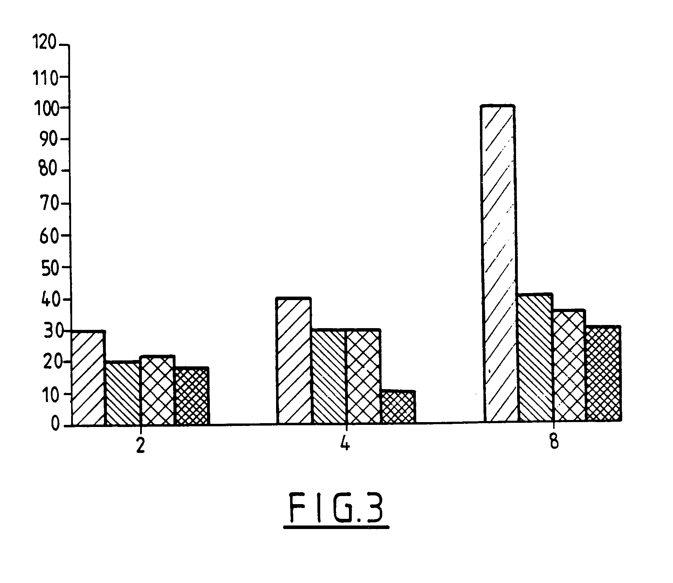

Les résultats obtenus sont illustrés sur la figure 3, qui comporte en abscisse le temps en jour et en ordonnée le rapport P/A. Le taux de rétraction est représenté par ▨ pour le témoin ; par ▧ pour le bFGF ; par ▨ pour les FGFs en présence d'héparane sulfate et par ▨ pour les FGFs associés aux dextrane fonctionnalisés.

Les résultats sont également présentés dans les tableaux IV et V ci-après ; le tableau IV donne le pourcentage de l'aire de cicatrisation en fonction de la quantité de dextrane fonctionnalisé (DF) en présence ou en l'absence de bFGF ; le tableau V donne le rapport P/A dans les mêmes conditions.

TABLEAU IV

| P/A | TEMOIN | DF 500 μg | DF 50 μg | DF 5 μg | bFGF 1 μg | bFGF 1 μg + DF à différentes concentrations : | | |
|-----|--------|-----------|----------|---------|-----------|-----|-----|-----|
| | | | | | | 500 | 50 | 5 |
| 2 j | 0,17 | 0,20 | 0,13 | 0,08 | 0,13 | 0,19 | 0,09 | 0,10 |
| 4 j | 0,20 | 0,18 | 0,19 | 0,11 | 0,15 | 0,13 | 0,11 | 0,08 |
| 8 j | 0,58 | 0,41 | 0,30 | 0,28 | 0,24 | 0,38 | 0,21 | 0,19 |

TABLEAU V

| | DF 500 $\mu$g | DF 50 $\mu$g | DF 5 $\mu$g | bFGF 1 $\mu$g | bFGF 1 $\mu$g + DF à différentes concentrations : | | |
|---|---|---|---|---|---|---|---|
| | | | | | 500$\mu$g | 50$\mu$g | 5$\mu$g |
| 2 j | 125 | 104 | 173 | 147 | 112 | 160 | 128 |
| 4 j | 105 | 213 | 160 | 169 | 182 | 231 | 260 |
| 8 j | 280 | 128 | 145 | 386 | 329 | 237 | 253 |

Les effets des dextranes fonctionnalisés sur cette rétraction sont particulièrement visibles aux quatrième et huitième jours après l'opération.

Les tableaux ci-dessus montrent bien l'effet cicatrisant propre des dextranes ; en effet, dans le tableau IV, après 8 jours, le pourcentage de l'aire en présence de 5 $\mu$g de DF est proche de celui en présence de 1 $\mu$g de bFGF seul, eux-mêmes inférieurs au témoin.

La rétraction est très faible en comparaison avec celles observées dans les expérimentations témoins ou celles observées en présence des FGFs seuls ou associés à des heparanes sulfates, conditions déjà nettement plus favorables que celles du témoin.

B) Etude histologique

Les régions traitées sont prélevées, fixées, imprégnées en paraffine. L'étude histologique est réalisée sur des coupes de 7 $\mu$m. Les colorations utilisées permettent des études topographiques et histochimiques.

L'analyse histologique montre que l'association FGF + DF accélère les étapes traditionnelles de la cicatrisation dermoépidermique et augmente la qualité des tissus reconstitués.

Le collagène imprégné permet une colonisation très rapide (1 jour) des catégories cellulaires environnantes (fibroblastes, musculaires lisses) à partir des tissus environnants sains, et en particulier à partir du conjonctif du plancher musculaire strié sous-jacent.

Dans le même temps, une néoangiogénèse permet au tissu en formation d'être colonisé par une densité très forte de capillaires sanguins. Au bout de trois jours (contre cinq à six pour les témoins), la réépithélialisation engagée à partir de l'épiderme des lèvres de la plaie arrive à affrontement. Le quatrième jour, l'épiderme est totalement reconstitué et les tissus sous-jacents, totalement réorganisés, présentent une densité normale comparativement aux témoins, à la densité beaucoup plus faible. Ces mêmes illustrations révèlent l'absence de rétraction des bords de la plaie pour les punchs traités par l'association FGF + dextranes fonctionalisés, contrairement aux témoins qui comblent, par extraction, les tissus excisés.

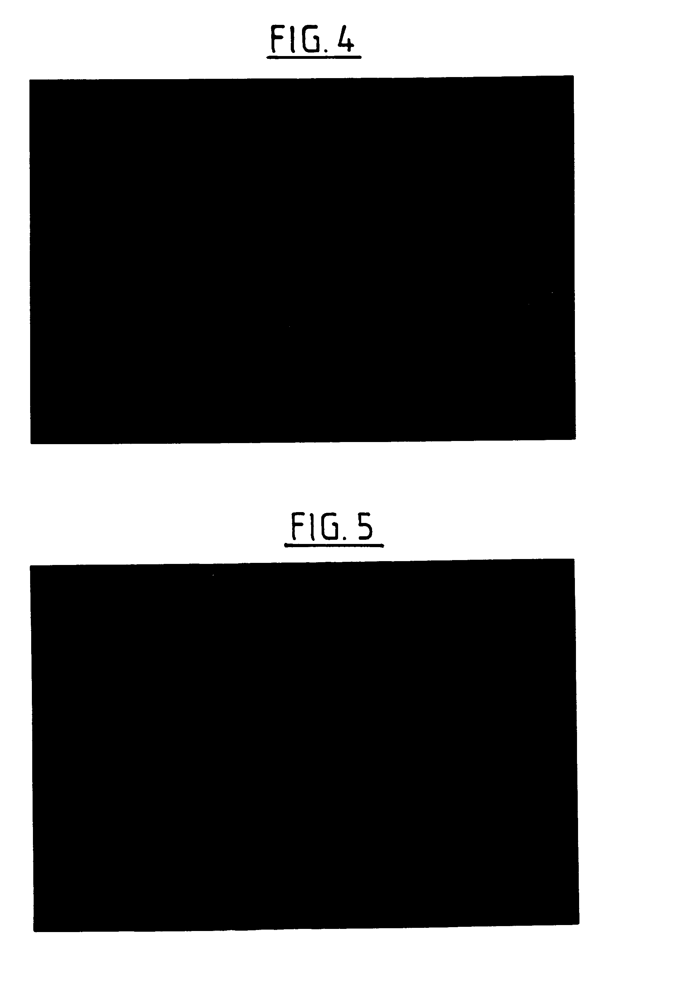

Les effets de l'association des bFGF et des dextranes fonctionnalisés sur la qualité de la cicatrisation, comparés à une cicatrisation naturelle sans adjonction de produits sont présentés sur les figures 4 et 5.

La figure 4 présente une photographie d'une coupe histologique d'une cicatrisation témoin (absence de traitement) quatre jours après la réalisation de la plaie (X 40). La figure 5 présente la photographie d'une coupe histologique d'une cicatrisation après traitement par un tampon de collagène imprégné d'une solution de bFGF à 1 $\mu$g/ml et de dextranes fonctionnalisés à 50 $\mu$g/ml, quatre jours après la réalisation de la plaie et au même grossissement de 40.

La figure 5 montre l'épiderme (E) reconstitué entièrement alors que sur la figure 4, il n'est pas reformé. Une rétraction des tissus environnants sur la plaie témoin n'est pas enregistrée sur la plaie traitée. Cet espace cicatriciel, relativement anarchique en figure 4, présente pour la plaie traitée (figure 5) une organisation et une densité cellulaire satisfaisante. Il se caractérise par la présence de vaisseaux sanguins traduisant l'effet angiogénique local de l'association des produits de cette invention.

Il apparaît donc que l'association bFGF + dextrane fonctionnalisé se présente comme un puissant agent cicatrisant *in vivo*, accélérant d'une part les processus naturels régénératifs, et permettant d'autre part une qualité accrue de la cicatrisation par l'absence de tout phénomène de rétraction comme la mobilisation rapide des différentes catégories cellulaires nécessaires à la restauration tissulaire.

**Exemple E : Etudes planimétriques et histologiques de l'effet cicatrisant de dextranes fonctionnalisés.**

Le protocole expérimental, en tous points identique à celui effectué dans le cadre de l'exemple D, est réalisé pour apprécier les effets cicatrisants des dextranes fonctionnalisés. Les dextranes étudiés sont répertoriés dans le tableau III de l'exemple C. Les effets cicatrisants de ces dextranes fonctionnalisés ou de leur association ont été appréciés par rapport à deux expérimentations témoins en présence de véhicule seul, de tampon de collagène, ou de tampon de collagène imprégné de dextrane non substitué (produit noté A).

A - Etude morphologique

L'observation de l'évolution des plaies à l'oeil nu permet de constater une action très nette des dextranes fonctionnalisés sur la vitesse et la qualité de la cicatrisation superficielle.

Par rapport aux expérimentations témoins, l'adhérence du véhicule est accélérée pour les plaies traitées par les dextranes fonctionnalisés.

La réépithélialisation suit une cinétique comparable à celle observée sous l'action des FGFs.

Le rapport P/A où P est le périmètre de la plaie et A la surface de la cicatrice traduit une diminution tout à fait significative du taux de rétraction cicatricielle. Les résultats obtenus sont illustrés par le tableau VI.

Ces expérimentations confirment le rôle spécifique des dextranes fonctionnalisés sur l'inhibition de la rétraction cicatricielle et la déformation du plan cutané environnant, comme cela a déjà été spécifié ci-dessus à l'exemple D.

B - Etude histologique

L'analyse est identique à celle menée dans l'exemple précédent.

Elle révèle par rapport aux observations des expérimentations témoins, une colonisation du collagène imprégné par les dextranes fonctionnalisés plus rapide et plus intense à partir des différents types cellulaires environnant la plaie.

La néoangiogénèse est nette mais moins soutenue que celle observée en présence des FGFs.

Les prolongements épidermiques s'affrontent aux alentours du jour 4, précédant d'au moins 24 heures la réépithélialisation observée chez les témoins.

Il apparaît donc un effet cicatrisant propre a l'action des dextranes fonctionnalisés qui se manifeste par une cicatrisation aux contours harmonieux traduisant une diminution de la contraction naturelle des berges de la plaie et une mobilisation accrue et rapide de cellules colonisatrices des collagènes aboutissant à un tissu de régénération plus dense et vascularisé que celui observé pour les expérimentations témoins. Un tel effet pourrait peut-être s'expliquer par le fait que les dextranes fonctionnalisés substitués potentialisent, au niveau des tissus, l'action des FGFs secrétés *in situ* par les tissus environnants.

TABLEAU VI

Les taux de rétraction P/A sont présentés pour les témoins collagènes seuls, collagène imprégné de dextrane de type A et pour les différents dextranes répertoriés précédemment. Toutes ces molécules agissent à une dilution de 3 μg/ml.

| P/A | 2 j | 4 j | 8 j |
|---|---|---|---|
| T(1) | 0,21 | 0,20 | 0,61 |
| T(2) | 0,20 | 0,25 | 0,55 |
| B | 0,16 | 0,18 | 0,30 |
| C | 0,15 | 0,18 | 0,26 |
| D | 0,13 | 0,13 | 0,20 |
| E | 0,14 | 0,16 | 0,22 |
| F | 0,10 | 0,11 | 0,20 |
| G | 0,15 | 0,18 | 0,29 |
| H | 0,13 | 0,14 | 0,30 |
| A+D | 0,18 | 0,20 | 0,28 |
| D+H | 0,11 | 0,15 | 0,22 |

(1) : Collagène

(2) : Collagène + A

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

**Revendications**
**Revendications pour les Etats contractants suivants : AT, BE, CH, DE, DK, FR, GB, IT, LI, LU, NL, SE**

1. Utilisation d'au moins un dextrane substitué fonctionnalisé par des fonctions choisies dans le groupe qui est constitué par le carboxyméthyle, le benzylamide et le benzylamide sulfonate, pour l'obtention d'un médicament destiné à une activité thérapeutique de régénération cellulaire et tissulaire.

2. Utilisation selon la revendication 1, caractérisée en ce que les dextranes substitués fonctionnalisés sont choisis dans le groupe qui comprend des dextranes solubles et des dextranes insolubles.

3. Composition stabilisée, caractérisée en ce qu'elle comprend un agent à activité régénératrice cellulaire et tissulaire selon l'une quelconque des revendications 1 et 2 associé avec au moins un FGF acide et/ou un FGF basique et/ou un dérivé et/ou un analogue et/ou un fragment de ceux-ci présentant une activité biologique, lequel agent est capable à la fois de restaurer partiellement ou totalement l'activité biologique du/des facteurs FGF acides et/ou basiques inactivés par un stockage de longue durée ou la température et de coopérer avec le FGF dans l'activité biologique de ce dernier.

4. Composition selon la revendication 3, caractérisée en ce que celle-ci comprend de 0,1 à 1 000 μg/ml d'au moins un agent à activité régénératrice cellulaire et tissulaire selon la revendication 1 ou la revendication 2 et de 0,01 ng/ml à 300 μg/ml d'au moins un FGF choisi dans le groupe constitué par les FGF acides, les FGF basiques, leurs dérivés, leurs analogues et leurs fragments présentant une activité biologique.

**5.** Composition contenant un agent à activité régénératrice cellulaire et tissulaire selon l'une quelconque des revendications 1 et 2 ou une composition contenant ledit agent associé à un FGF telle que définie dans la revendication 3 ou la revendication 4, caractérisée en ce qu'elle comprend, de plus, d'autres principes actifs associés.

**6.** Composition selon la revendication 5, caractérisée en ce que les principes actifs associés sont choisis dans le groupe qui comprend notamment les anesthésiques locaux, les antiinfectieux, des protéines sériques et du collagène.

**7.** Composition selon la revendication 5 ou la revendication 6, caractérisée en ce qu'elle comprend en outre au moins un véhicule pharmaceutiquement acceptable approprié et/ou un support physiologiquement acceptable.

**8.** Composition selon la revendication 7, caractérisée en ce que le véhicule est de l'eau et en ce que ladite composition comprend, en outre, des tampons et/ou des sels, de manière à maintenir le mélange approximativement à un pH compris entre 6,8 et 7,4 et à une force ionique comprise entre 0,1 et 0,2 en équivalent NaCl.

**9.** Composition selon l'une quelconque des revendications 5 à 8, caractérisée en ce qu'elle est incorporée à des liposomes appropriés.

**10.** Composition selon l'une quelconque des revendications 7 à 9, caractérisée en ce que le support est choisi dans le groupe qui comprend notamment les pansements et les biomatériaux.

**11.** Composition selon l'une quelconque des revendications 1 à 7, caractérisée en ce qu'elle est sous forme d'aérosol, lorsque le véhicule est un gaz approprié.

**12.** Composition selon l'une quelconque des revendications 1 à 9, caractérisée en ce qu'elle est incluse dans une forme galénique telle que pommade, crème, pâte, lotion ou imprègne un gel, notamment un gel de collagène.

**13.** Composition selon l'une quelconque des revendications 1 à 9, caractérisée en ce qu'elle est incluse et/ou imprègne un support approprié tel que pansement ou biomatériau favorisant directement ou indirectement la réparation cellulaire.

**Revendications pour l'Etat contractant suivant : ES**

**1.** Utilisation d'au moins un dextrane substitué fonctionnalisé par des fonctions choisies dans le groupe qui est constitué par le carboxyméthyle, le benzylamide et le benzylamide sulfonate, pour l'obtention d'un médicament destiné à une activité thérapeutique de régénération cellulaire et tissulaire.

**2.** Utilisation selon la revendication 1, caractérisée en ce que les dextranes substitués fonctionnalisés sont choisis dans le groupe qui comprend des dextranes solubles et des dextranes insolubles.

**3.** Procédé de préparation d'une composition stabilisée comprenant un dextrane substitué fonctionnalisé par des fonctions choisies dans le groupe qui est constitué par le carboxyméthyle, le benzylamide et le benzylamide sulfonate, à activité régénératrice cellulaire et tissulaire et au moins un FGF acide et/ou un FGF basique et/ou un dérivé et/ou un analogue et/ou un fragment de ceux-ci présentant une activité biologique, lequel dextrane est capable à la fois de restaurer partiellement ou totalement l'activité biologique du/des facteurs FGF acides et/ou basiques inactivés par un stockage de longue durée ou la température et de coopérer avec le FGF dans l'activité biologique de ce dernier, caractérisé en ce qu'il comprend :
a. la préparation d'un dextrane substitué fonctionnalisé (agent de régénération cellulaire et tissulaire) à partir de dextrane,
b. la préparation d'un FGF à partir d'extraits cellulaires et
c. le mélange du produit obtenu en a. et du produit obtenu en b.

4.  Procédé de préparation d'une composition selon la revendication 3, caractérisé en ce que la composition obtenue comprend de 0,1 à 1 000 µg/ml d'au moins un dextrane substitué fonctionnalisé à activité régénératrice cellulaire et tissulaire et de 0,01 ng/ml à 300 µg/ml d'au moins un FGF choisi dans le groupe constitué par les FGF acides, les FGF basiques, leurs dérivés, leurs analogues et leurs fragments présentant une activité biologique.

5.  Procédé de préparation d'une composition contenant un dextrane substitué fonctionnalisé ou d'une composition selon la revendication 3 ou la revendication 4, caractérisé en ce que l'on ajoute d'autres principes actifs associés.

6.  Procédé de préparation d'une composition selon la revendication 5, caractérisé en ce que les principes actifs associés sont choisis dans le groupe qui comprend notamment les anesthésiques locaux, les antiinfectieux, des protéines sériques et du collagène.

7.  Procédé de préparation d'une composition selon la revendication 5 ou la revendication 6, caractérisé en ce que l'on ajoute en outre au moins un véhicule pharmaceutiquement acceptable approprié et/ou un support physiologiquement acceptable.

8.  Procédé de préparation d'une composition selon la revendication 7, caractérisé en ce que le véhicule est de l'eau et en ce que ladite composition comprend, en outre, des tampons et/ou des sels, de manière à maintenir le mélange approximativement à un pH compris entre 6,8 et 7,4 et à une force ionique comprise entre 0,1 et 0,2 en équivalent NaCl.

9.  Procédé de préparation d'une composition selon l'une quelconque des revendications 5 à 8, caractérisé en ce que la composition obtenue est incorporée à des liposomes appropriés.

10. Procédé de préparation d'une composition selon l'une quelconque des revendications 7 à 9, caractérisé en ce que le support est choisi dans le groupe qui comprend notamment les pansements et les biomatériaux.

11. Procédé de préparation d'une composition selon l'une quelconque des revendications 5 à 10, caractérisé en ce que ladite composition est mise sous forme d'aérosol, lorsque le véhicule est un gaz approprié.

12. Procédé de préparation d'une composition selon l'une quelconque des revendications 5 à 11, caractérisé en ce que ladite composition est incluse dans une forme galénique telle que pommade, crème, pâte, lotion ou imprègne un gel, notamment un gel de collagène.

13. Procédé de préparation d'une composition selon l'une quelconque des revendications 5 à 11, caractérisé en ce que ladite composition est incluse et/ou imprègne un support approprié tel que pansement ou biomatériau favorisant directement ou indirectement la réparation cellulaire.

## Claims
### Claims for the following Contracting States : AT, BE, CH, DE, DK, FR, GB, IT, LI, LU, NL, SE

1.  Use of at least one functionalized substituted dextran containing functions selected from the group consisting of carboxymethyl, benzylamide and benzylamide sulphonate, for obtaining a drug intended for a therapeutic activity of cell and tissue regeneration.

2.  Use according to claim 1, characterized in that the functionalized substituted dextrans are selected from the group comprising soluble dextrans and insoluble dextrans.

3.  A stabilized composition, characterized in that it comprises an agent having a cell and tissue regenerating activity, according to any one of claims 1 and 2, in association with at least one acid FGF and/or one basic FGF and/or one derivative and/or one analogue and/or one fragment thereof having a biological activity which agent is capable, at the same time, of partially or totally restoring the biological activity of the acid and/or basic FGF/FGFs inactivated by prolonged storage or temperature and of cooperating with said FGF, in the biological activity of the latter.

4. A composition according to claim 3, characterized in that it comprises from 0.1 to 1 000 $\mu$g/ml of at least one agent having a cell and tissue regenerating activity, according to claim 1 or claim 2, and from 0.01 ng to 300 $\mu$g of at least one FGF selected from the group consisting of acid FGFs, basic FGFs and their derivatives, their analogues and their fragments having a biological activity.

5. A composition containing an agent having a cell and tissue regenerating activity, according to any one of claims 1 and 2, or a composition containing said agent in association with an FGF, as defined in claim 3 or claim 4, characterized in that it also comprises other associated active principles.

6. A composition according to claim 5, characterized in that the associated active principles are selected from the group comprising especially local anaesthetics, antiinfectious agents, serum proteins and collagen.

7. A composition according to claim 5 or claim 6, characterized in that it also comprises at least one pharmaceutically acceptable vehicle and/or a physiologically acceptable support.

8. A composition according to claim 7, characterized in that the vehicle is chosen is water and in that said composition also comprises buffers and/or salts so as to keep the mixture approximately at a pH of between 6.8 and 7.4 and at an ionic strength of between 0.1 and 0.2, in NaCl equivalents.

9. A composition according to any one of claims 5 to 8, characterized in that it is incorporated into appropriate liposomes.

10. A composition according to any one of claims 7 to 9, characterized in that the support is selected from the group comprising especially dressings and biomaterials.

11. A composition according to any one of claims 1 to 7, characterized in that it is in the form of an aerosol if the vehicle is an appropriate gas.

12. A composition according to any one of claims 1 to 9, characterized in that it is included in a medicinal form such as an ointment, cream, paste or lotion, or impregnated in a gel, especially a collagen gel.

13. A composition according to any one of claims 1 to 9, characterized in that it is included and/or impregnated in an appropriate support, such as dressing or biomaterial, which directly or indirectly favours cell repair.

**Claims for the following Contracting State : ES**

1. Use of at least one functionalized substituted dextran containing functions selected from the group consisting of carboxymethyl, benzylamide and benzylamide sulphonate, for obtaining a drug intended for a therapeutic activity of cell and tissue regeneration.

2. Use according to claim 1, characterized in that the functionalized substituted dextrans are selected from the group comprising soluble dextrans and insoluble dextrans.

3. Process of preparation of a stabilized composition, comprising a functionalized substituted dextran containing functions selected from the group consisting of carboxymethyl, benzylamide and benzylamide sulphonate, with cell and tissue regenerating activity and at least one acid FGF and/or one basic FGF and/or one derivative and/or one analogue and/or one fragment thereof having a biological activity, which dextran is capable, at the same time, of partially or totally restoring the biological activity of the acid and/or basic FGF/FGFs inactivated by prolonged storage or temperature and of cooperating with said FGF, in the biological activity of the latter, characterized in that it comprises:
a. the preparation of a functionalized substituted dextran from dextran,
b. the preparation of a FGF from cell extracts, and
c. the mixture of the product obtained in a. and the product obtained in b..

4. Process of preparation of a composition according to claim 3, characterized in that the obtained composition comprises from 0.1 to 1 000 $\mu$g/ml of at least one functionalized substituted dextran having a cell and tissue regenerating activity, and from 0.01 ng to 300 $\mu$g of at least one FGF selected from the group consisting of acid FGFs, basic FGFs and their derivatives, their analogues and their fragments having a biological activity.

5. Process of preparation of a composition containing a functionalized substituted dextran or a composition according to claim 3 or 4, characterized in that other active principles are added.

6. Process of preparation of a composition containing a functionalized substituted dextran or a composition according to claim 5, characterized in that the associated active principles are selected from the group comprising especially local anaesthetics, antiinfectious agents, serum proteins and collagen.

7. Process of preparation of a composition containing a functionalized substituted dextran or a composition according to claim 5 or 6, characterized in that at least one pharmaceutically acceptable vehicle and/or a physiologically acceptable support, is added.

8. Process of preparation of a composition containing a functionalized substituted dextran or a composition according to claim 7, characterized in that the vehicle is water and in that said composition also comprises buffers and/or salts so as to keep the mixture approximately at a pH of between 6.8 and 7.4 and at an ionic strength of between 0.1 and 0.2, in NaCl equivalents.

9. Process of preparation of a composition containing a functionalized substituted dextran or a composition according to any one of claims 5 to 8, characterized in that the obtained composition is incorporated into appropriate liposomes.

10. Process of preparation of a composition containing a functionalized substituted dextran or a composition according to any one of claims 7 to 9, characterized in that the support is selected from the group comprising especially dressings and biomaterials.

11. Process of preparation of a composition containing a functionalized substituted dextran or a composition according to any one of claims 1 to 7, characterized in that said composition is in the form of an aerosol if the vehicle is an appropriate gas.

12. Process of preparation of a composition containing a functionalized substituted dextran or a composition according to any one of claims 1 to 9, characterized in that said composition is included in a medicinal form such as an ointment, cream, paste or lotion, or impregnated in a gel, especially a collagen gel.

13. Process of preparation of a composition containing a functionalized substituted dextran or a composition according to any one of claims 1 to 9, characterized in that said composition is included and/or impregnated in an appropriate support, such as dressing or biomaterial, which directly or indirectly favours cell repair.

**Patentansprüche**

**Patentansprüche für folgende Vertragsstaaten : AT, BE, CH, DE, DK, FR, GB, IT, LI, LU, NL, SE**

1. Verwendung mindestens eines substituierten, durch die funktionellen Gruppen ausgewählt aus der Gruppe bestehend aus Carboxymethyl, Benzylamid und Benzylamidsulfonat funktionalisierten Dextrans zur Herstellung eines Arzneimittels für die therapeutische Wirkung einer Zell- und Gewebsregeneration.

2. Verwendung nach Anspruch 1, dadurch **gekennzeichnet**, daß die substituierten, funktionalisierten Dextrane aus der Gruppe bestehend aus löslichen Dextranen und unlöslichen Dextranen ausgewählt sind.

3. Stabilisiertes Präparat, dadurch **gekennzeichnet**, daß es ein Mittel mit zell- und gewebsregenerierender Wirkung nach einem der Ansprüche 1 und 2 zusammen mit mindestens einem sauren FGF und/oder einem basischen FGF und/oder einem Derivat und/oder einem Analogen und/oder einem Fragment davon, das eine biologische Aktivität besitzt, enthält, wobei das Mittel gleichzeitig in der Lage ist, die biologische Aktivität des/der sauren und/oder basischen, durch eine lange Lagerung oder durch

17

die Temperatur inaktivierten FGF-Faktoren ganz oder teilweise wiederherzustellen, und mit dem FGF bzgl. dessen biologischer Aktivität mitzuwirken.

4. Präparat nach Anspruch 3, dadurch **gekennzeichnet**, daß es 0,1 bis 1000 $\mu$g/ml mindestens eines Mittels mit zell- und gewebsregenerierender Wirkung nach Anspruch 1 oder Anspruch 2 und 0,01 ng/ml bis 300 $\mu$g/ml mindestens eines FGF ausgewählt aus der Gruppe bestehend aus sauren FGFs, basischen FGFs, ihren Derivaten, ihren Analogen und ihren Fragmenten, die eine biologische Aktivität besitzen, enthält.

5. Präparat enthaltend ein Mittel mit zell- und gewebsregenerierender Wirkung nach einem der Ansprüche 1 und 2, oder Präparat enthaltend das genannte Mittel zusammen mit einem FGF nach Anspruch 3 oder nach Anspruch 4, dadurch **gekennzeichnet**, daß es zusätzlich andere beigefügte Wirkstoffe enthält.

6. Präparat nach Anspruch 5, dadurch **gekennzeichnet**, daß die beigefügten Wirkstoffe aus der Gruppe, die insbesondere Lokalanästhetika, infektionsverhindernde Mittel, Serumproteine und Collagen umfaßt, ausgewählt sind.

7. Präparat nach Anspruch 5 oder 6, dadurch **gekennzeichnet**, daß es zusätzlich mindestens ein geeignetes, pharmazeutisch annehmbares Vehikel und/oder einen physiologisch annehmbaren Träger enthält.

8. Präparat nach Anspruch 7, dadurch **gekennzeichnet**, daß das Vehikel Wasser ist, und daß das Präparat zusätzlich Puffer und/oder Salze, um die Mischung ungefähr bei einem pH-Wert zwischen 6,8 und 7,4 und bei einer Ionenstärke zwischen 0,1 und 0,2, ausgedrückt als NaCl-Äquivalent, zu halten, enthält.

9. Präparat nach einem der Ansprüche 5 bis 8, dadurch **gekennzeichnet**, daß es in geeignete Liposomen eingearbeitet ist.

10. Präparat nach einem der Ansprüche 7 bis 9, dadurch **gekennzeichnet**, daß der Träger aus der Gruppe, die insbesondere Verbandsmaterialien und Biomaterialien umfaßt, ausgewählt ist.

11. Präparat nach einem der Ansprüche 1 bis 7, dadurch **gekennzeichnet**, daß es in Form eines Aerosols vorliegt, wenn das Vehikel ein geeignetes Gas ist.

12. Präparat nach einem der Ansprüche 1 bis 9, dadurch **gekennzeichnet**, daß es in einer galenischen Form, wie in einer Salbe, Creme, Paste, Lotion, eingeschlossen ist oder ein Gel, insbesondere ein Collagengel, tränkt.

13. Präparat nach einem der Ansprüche 1 bis 9, dadurch **gekennzeichnet**, daß es in einem geeigneten Träger, wie Verbandsmaterial oder Biomaterial, der direkt oder indirekt die Zellreparatur begünstigt, eingeschlossen ist und/oder ihn tränkt.

**Patentansprüche für folgenden Vertragsstaat : ES**

1. Verwendung mindestens eines substituierten, durch die funktionellen Gruppen ausgewählt aus der Gruppe bestehend aus Carboxymethyl, Benzylamid und Benzylamidsulfonat funktionalisierten Dextrans zur Herstellung eines Arzneimittels für die therapeutische Wirkung einer Zell- und Gewebsregeneration.

2. Verwendung nach Anspruch 1, dadurch **gekennzeichnet**, daß die substituierten, funktionalisierten Dextrane aus der Gruppe bestehend aus löslichen Dextranen und unlöslichen Dextranen ausgewählt sind.

3. Verfahren zur Herstellung eines stabilisierten Präparats, das ein substituiertes, durch die funktionellen Gruppen ausgewählt aus der Gruppe bestehend aus Carboxymethyl, Benzylamid und Benzylamidsulfonat funktionalisiertes Dextran mit zell- und gewebsregenerierender Wirkung und mindestens ein saures FGF und/oder ein basisches FGF und/oder ein Derivat und/oder ein Analoges und/oder ein Fragment

18

davon, das eine biologische Aktivität besitzt, enthält, wobei das Dextran gleichzeitig in der Lage ist, die biologische Aktivität des/der sauren und/oder basischen, durch eine lange Lagerung oder durch die Temperatur inaktivierten FGF-Faktoren ganz oder teilweise wiederherzustellen, und mit dem FGF bzgl. dessen biologischer Aktivität mitzuwirken, dadurch **gekennzeichnet**, daß es:

    a. die Herstellung eines substituierten, funktionalisierten Dextrans (Mittel zur Zell- und Gewebsregeneration) aus Dextran,

    b. die Herstellung eines FGF aus Zellextrakten und

    c. die Mischung des unter a. und unter b. erhaltenen Produkts

umfaßt.

4.    Verfahren zur Herstellung eines Präparats nach Anspruch 3, dadurch **gekennzeichnet**, daß das erhaltene Präparat 0,1 bis 1000 $\mu$g/ml mindestens eines substituierten, funktionalisierten Dextrans mit zell- und gewebsregenerierender Wirkung und 0,01 ng/ml bis 300 $\mu$g/ml mindestens eines FGF ausgewählt aus der Gruppe bestehend aus sauren FGFs, basischen FGFs, ihren Derivaten, ihren Analogen und ihren Fragmenten, die eine biologische Aktivität besitzen, enthält.

5.    Verfahren zur Herstellung eines Präparats, die ein funktionalisiertes, substituiertes Dextran enthält, oder einer Präparat nach Anspruch 3 oder Anspruch 4, dadurch **gekennzeichnet**, daß man andere Wirkstoffe zusetzt.

6.    Verfahren zur Herstellung eines Präparats nach Anspruch 5, dadurch **gekennzeichnet**, daß man die beigefügten Wirkstoffe aus der Gruppe, die insbesondere Lokalanästhetika, infektionsverhindernde Mittel, Serumproteine und Collagen umfaßt, auswählt.

7.    Verfahren zur Herstellung eines Präparats nach Anspruch 5 oder 6, dadurch **gekennzeichnet**, daß man zusätzlich mindestens ein geeignetes, pharmazeutisch annehmbares Vehikel und/oder einen physiologisch annehmbaren Träger zusetzt.

8.    Verfahren zur Herstellung eines Präparats nach Anspruch 7, dadurch **gekennzeichnet**, daß das Vehikel Wasser ist, und daß das Präparat zusätzlich Puffer und/oder Salze, um die Mischung ungefähr bei einem pH-Wert zwischen 6,8 und 7,4 und bei einer Ionenstärke zwischen 0,1 und 0,2, ausgedrückt als NaCl-Äquivalent, zu halten, enthält.

9.    Verfahren zur Herstellung eines Präparats nach einem der Ansprüche 5 bis 8, dadurch **gekennzeichnet**, daß man das erhaltene Präparat in geeignete Liposomen einarbeitet.

10.   Verfahren zur Herstellung eines Präparats nach einem der Ansprüche 7 bis 9, dadurch **gekennzeichnet**, daß man den Träger aus der Gruppe, die insbesondere Verbandsmaterialien und Biomaterialien umfaßt, auswählt.

11.   Verfahren zur Herstellung eines Präparats nach einem der Ansprüche 5 bis 10, dadurch **gekennzeichnet**, daß man das Präparat als Aerosol formuliert, wenn das Vehikel ein geeignetes Gas ist.

12.   Verfahren zur Herstellung eines Präparats nach einem der Ansprüche 5 bis 11, dadurch **gekennzeichnet**, daß man das Präparat in eine galenische Form, wie eine Salbe, Creme, Paste, Lotion, einschließt oder ein Gel, insbesondere ein Collagengel, damit tränkt.

13.   Verfahren zur Herstellung eines Präparats nach einem der Ansprüche 5 bis 11, dadurch **gekennzeichnet**, daß man das Präparat in einen geeigneten Träger, wie Verbandsmaterial oder Biomaterial, der die Zellreparatur direkt oder indirekt begünstigt, einschließt oder diesen damit tränkt.

FIG.1

FIG.2

FIG.3

## FIG. 4

## FIG. 5